# EUROPEAN PATENT SPECIFICATION

(11) **EP 2 283 025 B1**
(45) Date of publication and mention of the grant of the patent: **07.12.2011**
(21) Application number: 09721251.8
(22) Date of filing: 19.03.2009
(51) Int. Cl.: C07J 1/00, A01N 45/00

(54) **5-FLUORO-3ALPHA ,17BETA-DIHYDROXY-5ALPHA-ANDROSTAN-6-ONE, THE METHOD FOR ITS PRODUCTION AND ITS USE FOR INFLUENCING PLANT GROWTH**
5-FLUOR-3ALPHA,17BETA-DIHYDROXY-5ALPHA-ANDROSTAN-6-ON, VERFAHREN ZU DESSEN HERSTELLUNG UND DESSEN VERWENDUNG ZUR BEEINFLUSSUNG VON PFLANZENWACHSTUM
5-FLUORO-3ALPHA ,17BETA-DIHYDROXY-5ALPHA-ANDROSTAN-6-ONE, PROCEDE DE PRODUCTION CORRESPONDANT, ET SON UTILISATION POUR AGIR SUR LA CROISSANCE DE PLANTES

(30) Priority: 20.03.2008 CZ 20080183
(43) Date of publication of application: 16.02.2011
(73) Proprietor: Ustav Organicke Chemie a Biochemie Akademie Ved Ceske Republiky, V.V.I., 166 10 Praha 6 (CZ)
(72) Inventor: SLAVIKOVA, Barbora, 252 28 Cernosice (CZ); KASAL, Alexander, 101 00 Praha 10 (CZ); KOHOUT, Ladislav, 163 00 Praha 6 (CZ); BUDESINSKY, Milos, 182 00 Praha 8 (CZ); SWACZYNOVA, Jana, 779 00 Olomouc (CZ)
(74) Representative: Herman, Vaclav
(86) International application number: PCT/CZ2009/000040
(87) International publication number: WO 2009/115060

(56) References cited:
- WO-A-03/003834
- SLAVIKOVA, BARBORA ET AL: "Brassinosteroids: Synthesis and Activity of Some Fluoro Analogues" JOURNAL OF MEDICINAL CHEMISTRY , 51(13), 3979-3984 CODEN: JMCMAR; ISSN: 0022-2623, 2008, XP002531611
- GAUDINOVA A ET AL: "DIFFERENT EFFECTS OF TWO BRASSINOSTEROIDS ON GROWTH, AUXIN AND CYTOKININ CONTENT IN TOBACCO CALLUS TISSUE" PLANT GROWTH REGULATION, DORDRECHT, vol. 17, no. 2, 1 January 1995 (1995-01-01), pages 121-126, XP008010568 ISSN: 0167-6903

## Description

### Field of the Invention

The invention relates to 5-fluoro-3α,17β-dihydroxy-5α-androstan-6-one, the method for its production and its use for influencing of plant growth

### Background of the Invention

Various plant hormones and their mixtures are used in order to stimulate plant growth. Considerable interest has been given to brassinosteroids, i.e., analogues of natural plant hormone brassinolide, (22*R*,23*R*,24*S*)-2α,3α,22,23-tetrahydroxy-24-methyl-7a-homo-7-oxa-5α-cholestan-6-one (**I**) 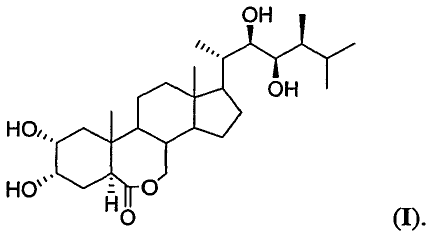 This compound is isolated from plant material, which is unacceptable for practical use because brassinolide exists in very low concentrations in the nature. For instance, rape pollen only contains 0.1 ppm [Groves M. D. et al.: Nature (London) 281, 216 (1979).], other plant species contain even less [0,000 003 ppm in celery cabbage: Morishita T. et al.: Phytochemistry 22, 1051 (1983)]. Brassinolide also can be obtained by chemical synthesis, which is unrealistic too because of a complicated multi step procedure with low yields [e.g., Fung et al., J. Amer. Chem. Soc. 102, 6580 (1980), Hazra B.G. et al.: Tetrahedron 50, 2523 (1994), Koreeda M. et al.: Synlett 8 650 (1995) and others]. Thus, 24-epibrassinolide, (22*R*,23*R*,24*R*)-2α,3α,22,23-tetrahydroxy-24-methyl-7a-homo-7-oxa--5α-cholestan-6-one (II) is being used both in research and in the field. 24-Epibrassinolide (II), 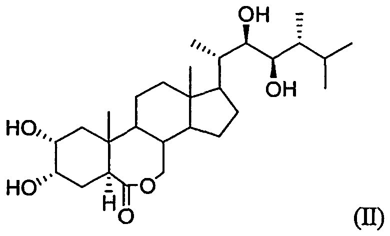 a brassinolide isomer, differs from brassinolide just by configuration of the methyl group in position 24. The synthesis of this compound is rather complicated too, even though the synthesis was substantially reduced recently [see e.g., Kohout L.: Coll. Czech. Chem. Commun. 59, 457 (1994), Brosa C. et al.: Tetr. Lett. 32, 7057 (1992) and Khripach V.A. et al.: Zh. Org. Khim. 30, 1650 (1994)].

New types of brassinosteroids are being prepared and used [e.g., Khripach V.A. et al.: Vesti Akad. Nauk. Belarusi, Ser. Khim. Nauk (3), 64 (1995) or Mori K. et al.: Liebigs Ann. Chem. 815 (1988)]. These compounds have a low activity, their synthesis is not efficient, too complicated and expensive, thus the above mentioned 24-epibrassinolide (II) remains the most often used brassinosteroid. The drawback of its use was discussed above.

A few years ago, the synthesis and use of a new brassinosteroid was published: 2α,3α,17β-trihydroxy-5α-androstan-6-one (III) 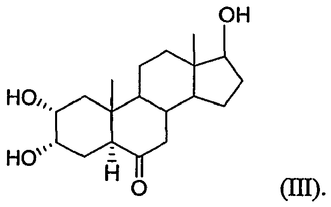 Its cost is much lower than that of the above given natural brassinosteroids (Kohout L., Kasal A., Chodounská H.: The use of 2α,3α,17β-trihydroxy-5α-androstan-6-one for treatment of the stress in plants, CZ 294 343 (4.10.2004), and Kohout L., Kasal A., Chodounská H., Slavíková B., Hnili 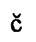 ková J.: EP 1 401 278 (28. 9. 2005), Byelorussia patent No. 9385 (05.03.2007) a Ukrainian patent 74670 (16.01.2006),
Gaudinová et al. Plant Growth regulation 17: 121-126 (1995).

We have now found that our new product, 5-fluoro-3α,17β-dihydroxy-5α-androstan-6-one (IV) 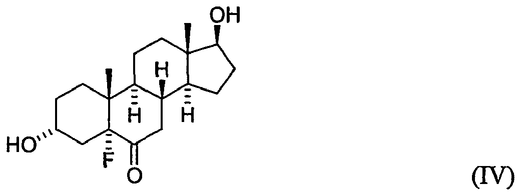 is surprisingly potent plant growth regulator. Its synthesis is faster and much more cost effective when compared with natural brassinosteroids but also with the above given synthetic brassinosteroid III.

### Detailed description

The invention relates to 5-fluoro-3α,17β-dihydroxy-5α-androstan-6-one (IV)

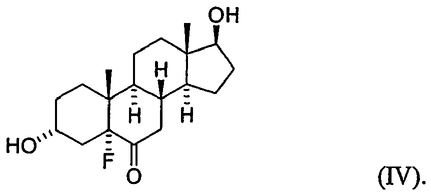

It also relates to the synthesis of 5-fluoro-3α,17β-dihydroxy-5α-androstan-6-one (IV) 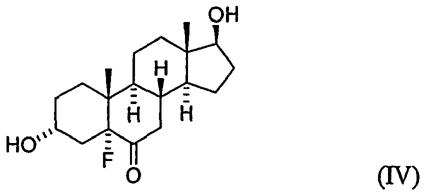 which consists in a partial hydrolysis of the known 5-fluoro-6-oxo-5α-androstane-3β,17β-diyl diacetate V 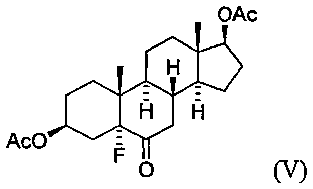 with a mild alkaline reagent, preferably with one equivalent of potassium carbonate, in organic solvent such as alcohol or aqueous alcohol, preferably aqueous methanol, under the formation of 5-fluoro-3β-hydroxy-6-oxo-5α-androstan-17β-yl acetate VI

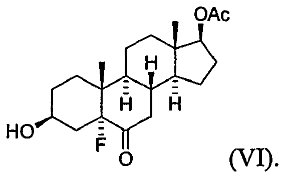

In this mono acetate VI, the configuration of the hydroxyl group in position 3 has to be changed. This can be achieved directly under conditions of the Mitsunobu reaction, or indirectly, after conversion into a suitable reactive intermediate such as tosylate or mesylate, using sodium nitrite in a suitable solvent such as DMAA, HMPA, DMF, DMSO and the like, preferably in HMPA. The corresponding 3α-alcohol is formed, 5-fluoro-3α-hydroxy-6-oxo-5α-androstan-17β-yl acetate VII

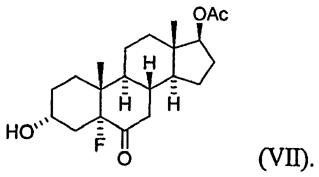

Hydrolysis of the protecting acetoxy group in alkaline medium, such as alkali carbonate or hydroxide, for instance sodium or potassium carbonate or hydroxide, or in acidic medium, such as sulphuric, hydrochloric, hydrobromic acid and the like, in suitable solvent, preferably in methanol, yields the target compound, 5-fluoro-3α,17β-dihydroxy-5α-androstan-6-one IV

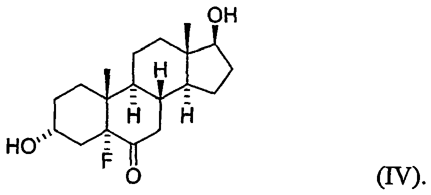

This invention further relates to the use of 5-fluoro-3α,17β-dihydroxy-5α-androstan-6-one (IV) for the stimulation of plant growth in a similar way as natural and known synthetic brassinosteroids act. A concentrated solution of this compound in a suitable solvent such as alcohol, e.g., ethanol, propanol or isopropanol, or inert solvent such dimethylsulfoxide, dimethylformamide, N,N-dimethylacetamide and so on. For application on the plants, the concentrate is then diluted with water to get suitable concentration, e.g., 10⁻⁶ M. It is also possible to dissolve the compound directly in the final volume suitable for application. The application can be done in any suitable way, e.g., using a hand-held spray gun, a shoulder-held spray or any large-scale industrial spray. There is no limit for the choice of the spraying device because the concentration of the active compound is very low. Surface activity can be increased by using surfactant, such as Tween, e.g. Tween 20.

A solution prepared in this way is useful for the application, when a higher plant growth or protection against stress are to be achieved, for instance in drought, lack of nutrients, too low or too high temperature, or the presence of stressing compounds such as excess of salt, pesticides or herbicides and so on.

The Invention will be illustrated in detailes on the following examples, which in no way restrict the scope of the Invention

### EXAMPLES

### Example 1

### The synthesis of 5-fluoro-3β-hydroxy-6-oxo-5α-androstan-17β-yl acetate (compound VI)

A solution of potassium hydroxide (670 mg, 4,85 mmol) in water (12 ml) and methanol (24 ml) is added to a solution of 5-fluoro-6-oxo-5α-androstane-3β,17β-diyl diacetate (1.83 g, 4.48 mmol) in methanol (180 ml). After one hour at room temperature acetic acid (0,6 ml) is added and the solution is concentrated *in vacuo.* A white solid, precipitated on addition of brine, is extracted with ethyl acetate. The extract is washed with brine, dried and stripped of the solvent *in vacuo.* Chromatography of the remainder on a column of silica gel (150 ml) yields alcohol VI (1.25 g, 76 %). M.p. 165-167 °C (1.18 g on crystallization from acetone-heptane); [α]_{D} -16.4 (c 0.18). IR spectrum: 3500, 3439, 3611, 1048 (OH), 1725, 1257 (COCH₃).

### Example 2

### The synthesis of 5-fluoro-3α-hydroxy-6-oxo-5α-androstan-17β-yl acetate (VII)

4-Toluenesulfonyl chloride (200 mg, 1.04 mmol) is added to a solution of 3β-alcohol, VI (200 mg, 0.55 mmol) in pyridine (2 ml). After 48 h, the mixture is diluted with brine, a white precipitate is extracted with chloroform, the extract is washed with water, dilute hydrochloric acid, water, a solution of sodium hydrogen carbonate, water and dried with anhydrous sodium sulfate. After stripping of the solvents *in vacuo,* tosylate is obtained which is then is solvolysed with sodium nitrite (480 mg, 0.70 mmol) in HMPA (25 ml) at 90 °C for 2 h under nitrogen. The mixture is poured into water and the product extracted with chloroform. The extract is then subsequently washed with water, dilute hydrochloric acid, water, a solution of potassium hydrogen carbonate and water. The extract is concentrated in vacuum and applied on PLC plates (5 plates, benzene/ethyl acetate, 7:3), yielding white crystals of the 3α alcohol VII (83 mg, 41 %). M.p. 139-142 °C (acetone/heptane). [α]_{D} -13.6 (c 0.21). IR spectrum: 3617, 1017 (OH), 1725 (AcO). ¹H NMR: δ 0.78 (s, 3H, H-18); 0.80 (s, 3H, H-19); 2.04 (s, 3H, CH₃CO); 4.08 (m, 1H, *W∼* 16 Hz, H-3); 4.64 (t, 1H, *J=*8.5 Hz, H-17).

### Example 3

### The synthesis of 5-fluoro-3α,17β-dihydroxy-5α-androstan-6-one (IV)

5-Fluoro-3α-hydroxy-6-oxo-5α-androstan-17β-yl acetate VII, prepared according to example 2 (70 mg, 0.19 mmol) in chloroform (0.5 ml) is hydrolysed by addition of a solution of hydrochloric acid (0.1 ml) in methanol (5 ml). The reaction mixture is kept at room temperature for 20 h. A solution of potassium carbonate is added and the product is extracted with chloroform. The extract is washed with brine, dried and concentrated in *vacuo*. Chromatography of the product using two PLC plates yields white crystals of the target 5-fluoro-3α,17β-dihydroxy-5α-androstan-6-one (50 mg, 81 %). M.p. 260-262 °C (acetone/heptane). [α]_{D}-12.4 (c 0.31). IR spectrum: 3617, 1070 (OH), 1725 (C=O), 1020 (C-F). ¹H NMR: δ 0.74 (s, 3H, H-18); 0.80 (s, 3H, H-19); 3.70 (t, 1H, *J*=8.5 Hz, H-17); 4.08 (m, 1H, *W∼* 16 Hz, H-3). For C₁₉H₂₉FO₃ (324,4) calculated: 70.34 % C, 9.01 % H, found: 70.13 % C, 8.83 % H.

### Example 4

### The preparation of the solution of compound IV for application in plants

5-Fluoro-3α,17β-dihydroxy-5α-androstan-6-one (5 mg) is added to 10 ml of isopropanol. The solution is then diluted with water to give a suitable concentration.

### Example 5

### The preparation of the solution of compound IV and a surfactant for application in plants

5-Fluoro-3α,17β-dihydroxy-5α-androstan-6-one (5 mg) and Tween 20 (1 ml) are added to 10 ml of ethanol. The solution is then diluted with water to give a suitable concentration.

### Example 6

### The preparation of an aqueous solution of compound IV for application in plants

5-Fluoro-3α,17β-dihydroxy-5α-androstan-6-one (0.2 mg) is added to 101 of water. The solution is ready for application.

### Example 7

### Bean Second Internode Bioassay

Bean seeds (Phaseolus vulgaris L., cv. Pinto) were germinated for two days. Selected germinated seeds were planted into pots containing perlite and a modified Hoagland's solution (half concentration, pH 5.7). The pots were placed in a light-controlled cultivation room (25 to 27 °C, light: 48 W/m², light/dark period: 16 h/8 h). Groups of eight 7-day-old bean seedlings with 2 mm long second internodes were treated with different amounts of the tested compounds in fractionated lanolin (5 µl). Tested compound was applied as micro drop to the place after removed bract at the beginning of the second internode. The control plants were treated with lanolin alone. At each experiments at least seven plants were used and the test was repeated three times. The measurements (the length of the second internode) were taken after 5 days. The difference in the length of the second internode of the treated and control plants was used as a measure of the activity.

| Tested compound | Elongation (mm) of the second internode at concentration 10⁻¹⁰ mol.l⁻¹ | SD |
|---|---|---|
| 24-epiBR | 54 | ±5.4 |
| IV | 43.4 | ±0.9 |

Above given results show that 5-fluoro-3α,17β-dihydroxy-5α-androstan-6-one is very near with its activity to activity of natural brassinosteroid, 24-epibrassinolide.

### Example 8

### Conservation of green and dry mass of beans

The application solution A is made by adding of 0.01 ml of a solution prepared according to the example 5 to 500 ml of water. Bean plants planted in the pots in greenhouse at the beginning of their second internode stage (length of internode 2 mm) are divided to 6 groups each of the same number of plants. The first and the second groups are watered with water containing 1 % solution of sodium chloride, the third and the fourth groups are planted in greenhouse at temperature 15°C, the fifth and the sixth groups are planted under standard condition. The second, the fourth and the sixth groups are sprayed with the application solution A. After 4 days green masses of all groups are recorded, left to dry and again recorded.

**Table 1**

| Green and dry masses of bean plants (in % to control, i.e. group 5 =100 %) after 4 days of growth | | | |
|---|---|---|---|
| | application | green mass | dry mass |
| group 1 | NaCl | 72 | 74 |
| group 2 | NaCl + solution A | 110 | 113 |
| group 3 | 15°C | 71 | 76 |
| group 4 | 15°C + solution A | 112 | 110 |
| group 5 | 0 | 100 | 100 |
| group 6 | 0 + solution A | 127 | 130 |

Table 1 shows that the use of the application solution A increases green and dry masses in all cases in comparison with control and that in all cases pronounced removal of stress influence to plants occured.

## Claims

1. 5-Fluoro-3α,17β-dihydroxy-5α-androstan-6-one IV

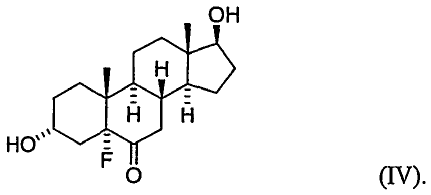

2. A method for production of 5-fluoro-3α,17β-dihydroxy-5α-androstan-6-one IV 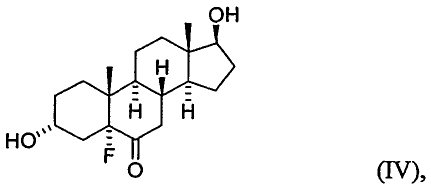 comprising partial hydrolyses of the known 5-fluoro-6-oxo-5α-androstan-3β,17β-diyl diacetate V 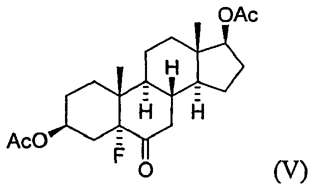 with mild alkaline agent in organic solvent to give 5-fluoro-3β-hydroxy-6-oxo-5α-androstan-17β-yl acetate VI 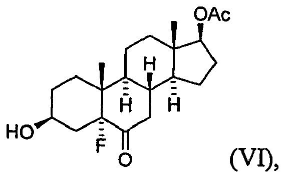 in which the change of hydroxyl configuration at the position 3 is made converting it to a solvolysable derivative and this derivative is solvolysed to afford 5-fluoro-3a-hydroxy-6-oxo-5α-androstan-17β-yl acetate VII 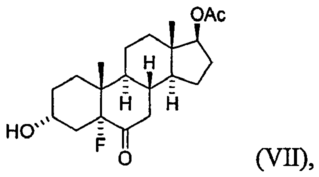 which on hydrolyses afforded desired 5-fluoro-3α,17β-dihydroxy-5α-androstan-6-one IV

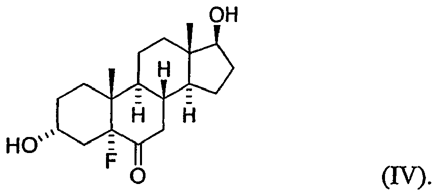

3. The method of the Claim 2, wherein hydrolyses of compound V is carried out with one equivalent of potassium carbonate in methanol.

4. The method of the Claim 2 or 3, wherein the configuration change is carried out by solvolyses of tosyloxy derivative with sodium nitrite in HMPA.

5. A use of 5-fluoro-3α,17β-dihydroxy-5α-androstan-6-one IV 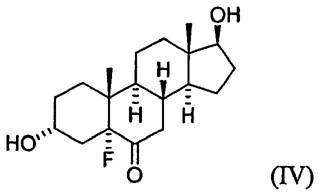 for plant growth regulation.

6. The use of claim 5 for stimulation of plant growth.

7. The use of claim 5 for removal of plant stress.

## Patentansprüche

1. 5-Fluor-3α,17β-dihydroxy-5α-androstan-6-on der Formel IV

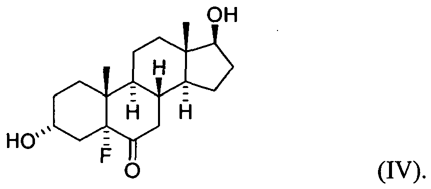

2. Verfahren zur Herstellung von 5-Fluor-3α,17β-dihydroxy-5α-androstan-6-on der Formel IV 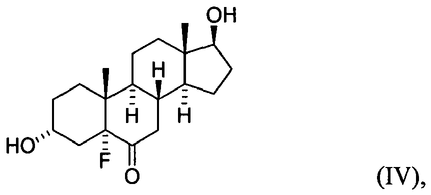 **dadurch gekennzeichnet, dass** das bekannte 5-Fluor-6-oxo-5α-androstan-3β,17β-diyl Azetat der Formel V 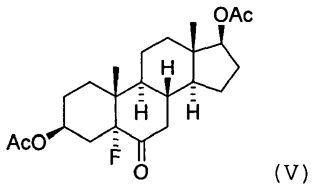 mit einem milden alkalischen Mittel im organischen Lösungsmittel unter Entstehung von 5-Fluor-3β-hydroxy-6-oxo-5α-androstan-17β-yl Azetat der Formel VI 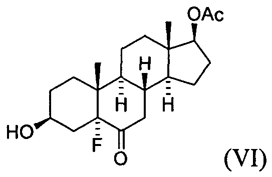 partiell hydrolysiert wird, wobei sich die Konfiguration des Hydroxyls in Position 3 so verändert, dass die Verbindung zu einem solvolysierbaren Derivat wird, durch dessen Solvolyse 5-Fluor-3α-hydroxy-6-oxo-5α-androstan-17β-yl Azetat der Formel VII 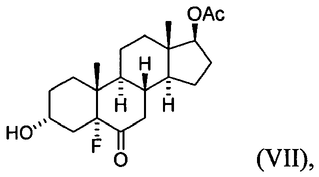 ensteht, nach dessen Hydrolyse man den verlangten 5-Fluor-3α,17β-dihydroxy-5α-androstan-6-on der Formel IV 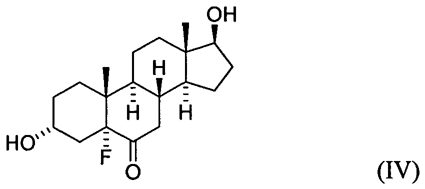 bekommt.

3. Verfahren nach Anspruch 2, **dadurch gekennzeichnet, dass** die Hydrolyse der Verbindung V mittels eines Äquivalents von Kaliumkarbonat in Methanol durchgeführt wird.

4. Verfahren nach Anspruch 2 oder 3, **dadurch gekennzeichnet, dass** die Konfigurationsänderung mittels Solvolyse des Tosylderivates mit Natriumnitrit in HMPA durchgeführt wird.

5. Benützung des 5-Fluor-3α,17β-dihydroxy-5α-androstan-6-on der Formel IV 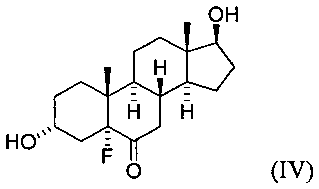 für die Regulierung des Pflanzenwachstums.

6. Benützung nach Anspruch 5 für die Stimulierung des Pflanzenwachstums.

7. Benützung nach Anspruch 5 für die Beseitigung von Pflanzenstress.

## Revendications

1. 5-Fluoro-3α,17β-dihydroxy-5α-androstan-6-one IV

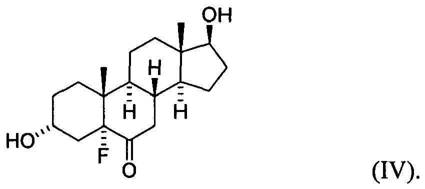

2. Procédé pour la production de 5-fluoro-3α,17β-dihydroxy-5α-androstan-6-one IV 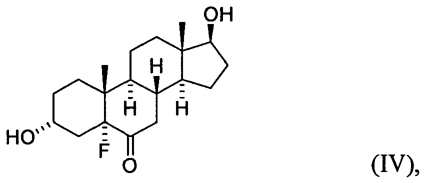 comprenant une hydrolyse partielle du diacétate de 5-fluoro-3α,17β-dihydroxy-5α-androstan-3β,17β-diyle V connu 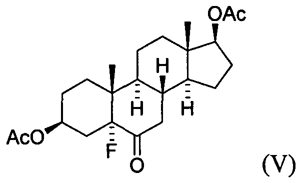 par l'agent alkalin doux dans le solvant organique a donné l'acétate de 5-fluoro-3β-hydroxy-6-oxo-5α-androstan-17β-yle VI 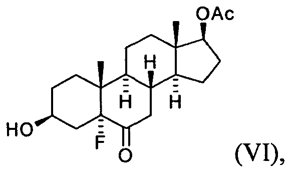 dont la configuration d'hydroxyle à la position 3 est changée par la convertion au derivé solvolysable et ce derivé est solvolysé pour fournir l'acétate de 5-fluoro-3α-hydroxy-6-oxo-5α-androstan-17β-yle VII 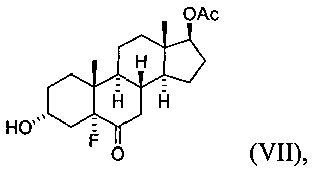 par une hydrolyse duquel la 5-fluoro-3α,17β-dihydroxy-5α-androstan-6-one IV 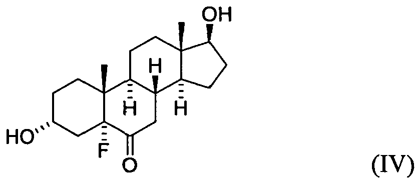 desirée est obtenue.

3. Procédé selon la revendication 2, dans lequel l'hydrolyse s'effectue avec un équivalent de carbonate de potassium dans le méthanol.

4. Procédé selon la revendication 2 ou 3, dans lequel le changement de configuration s'effectue par une solvolyse du derivé de tosyloxy par nitrite de sodium dans HMPA.

5. Utilisation de 5-fluoro-3α,17β-dihydroxy-5α-androstan-6-one IV 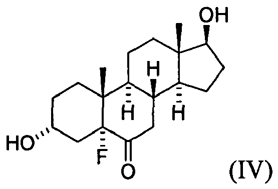 pour régler la croissance des plantes.

6. Utilisation selon la reventication 5 pour stimuler la croissance de plantes.

7. Utilisation selon la reventication 5 pour supprimer le stress de plantes.
